# EUROPÄISCHE PATENTANMELDUNG

(11) **EP 4 786 975 A1**
(43) Veröffentlichungstag der Anmeldung: **05.08.2026**
(21) Anmeldenummer: 26154361.5
(22) Anmeldetag: 27.01.2026
(51) Int. Cl.: G01N 33/36

(54) **VERFAHREN ZUR BESTIMMUNG EINES EIGENSCHAFTSWERTS MINDESTENS EINES KERNFADENS, VERFAHREN ZUM BETRIEB EINER SPINNVORRICHTUNG, VORRICHTUNG ZUR DATENVERARBEITUNG, FADENSENSOREINRICHTUNG, SPINNVORRICHTUNG UND COMPUTERPROGRAMM**

(30) Priorität: 03.02.2025 LU 509834
(71) Anmelder: Saurer Spinning Solutions GmbH & Co. KG, 52531 Übach-Palenberg (DE)
(72) Erfinder: Spitzer, Michael, 52156 Monschau-Kalterherberg (DE); Küppers, Dr. Simon, 70439 Stuttgart (DE)
(74) Vertreter: Morgenthum-Neurode, Mirko

(57) **Zusammenfassung**

Die Erfindung betrifft ein Verfahren zur Bestimmung eines Eigenschaftswerts, insbesondere eines Werkstoffs und/oder einer Feinheit, mindestens eines Kernfadens. Um den Eigenschaftswert zu bestimmen, wird ein Erfassungswert, der für mindestens einen Kraft-Dehnungs-Wert des Kernfadens spezifisch ist, empfangen und ausgewertet. Ferner betrifft die Erfindung ein Verfahren zum Betrieb einer Spinnvorrichtung, eine Vorrichtung zur Datenverarbeitung, eine Fadensensoreinrichtung, eine Spinnvorrichtung und ein Computerprogramm.

## Beschreibung

Die vorliegende Erfindung betrifft Verfahren zur Bestimmung eines Eigenschaftswerts mindestens eines Kernfadens, ein Verfahren zum Betrieb einer Spinnvorrichtung, eine Vorrichtung zur Datenverarbeitung, eine Fadensensoreinrichtung, eine Spinnvorrichtung und ein Computerprogramm.

### Stand der Technik

Bei der Herstellung von Spinnfäden, die Kernfäden umfassen, ist es bevorzugt, Eigenschaften der Kernfäden zu prüfen, um die Qualität der Spinnfäden sicherzustellen.

Aus dem Stand der Technik sind Verfahren und Vorrichtungen zur Prüfung von Kernfäden bekannt, die teilweise äußerst aufwendig oder ungenau sind.

Die Druckschriften CN 207891478 U, DE 10 2007 057 920 A1, EP 3 040 458 B1, EP 3 802 926 B1 und IN 2022 140 080 10 A offenbaren Verfahren und Vorrichtungen zur Prüfung von Fäden.

### Offenbarung der Erfindung

Eine Aufgabe der Erfindung ist es, eine alternative oder bessere technische Lösung zur Prüfung von Fäden, insbesondere Kernfäden, bereitzustellen, die vorzugsweise die Nachteile aus dem Stand der Technik überwindet.

Die voranstehende Aufgabe wird gelöst durch ein Verfahren zur Bestimmung eines Eigenschaftswerts mindestens eines Kernfadens, ein Verfahren zum Betrieb einer Spinnvorrichtung, eine Vorrichtung zur Datenverarbeitung, eine Fadensensoreinrichtung, eine Spinnvorrichtung sowie ein Computerprogramm mit den Merkmalen der entsprechenden unabhängigen Ansprüche. Weitere Merkmale und Details der Erfindung ergeben sich aus den jeweiligen Unteransprüchen, der Beschreibung und den Zeichnungen. Dabei gelten Merkmale und Details, die im Zusammenhang mit dem erfindungsgemäßen Verfahren zur Bestimmung eines Eigenschaftswerts beschrieben sind, selbstverständlich auch im Zusammenhang mit dem erfindungsgemäßen Verfahren zum Betrieb einer Spinnvorrichtung, der erfindungsgemäßen Vorrichtung zur Datenverarbeitung, der erfindungsgemäßen Fadensensoreinrichtung, der erfindungsgemäßen Spinnvorrichtung und dem erfindungsgemäßen Computerprogramm, und jeweils umgekehrt, sodass bezüglich der Offenbarung zu den einzelnen Erfindungsaspekten stets wechselseitig Bezug genommen wird bzw. werden kann.

Das erfindungsgemäße Verfahren zur Bestimmung eines Eigenschaftswerts mindestens eines Kernfadens weist die nachfolgenden Schritte auf, welche vorzugsweise nacheinander und/oder zumindest teilweise zeitlich parallel, und außerdem bevorzugt automatisiert, ausgeführt werden:
- Empfangen von mindestens einem Erfassungswert, wobei der mindestens eine Erfassungswert für mindestens einen Kraft-Dehnungs-Wert des Kernfadens spezifisch sein kann,
- Auswerten des mindestens einen Erfassungswerts (Auswertung),
- Bestimmen des Eigenschaftswerts, insbesondere des Werkstoffs des Kernfadens und/oder der Feinheit, bevorzugterweise des Durchmessers, des Kernfadens, auf Basis der Auswertung.

Dies hat bspw. die Wirkung, dass sichergestellt wird, dass der Kernfaden hinsichtlich des Materials oder Feinheitsgrades der geplanten Produktion einer Arbeitsstelle einer Faden herstellenden Textilmaschine entspricht. Es kann ferner sichergestellt werden, dass ggf. aufgetretene Systemfehler der Textilmaschine hinsichtlich eines Transportes und einer Zuordnung des Kernfadens vor dem Produktionsstart der Arbeitsstelle erkannt werden. Hierdurch kann eine alternative oder bessere technische Lösung zur Prüfung von Fäden, insbesondere Kernfäden, bereitgestellt werden, die insbesondere die Nachteile aus dem Stand der Technik überwindet. Mit anderen Worten handelt es sich vorzugsweise bei dem erfindungsgemäßen Verfahren um eine Untersuchung oder Prüfung des Kernfadens.

Insbesondere hat die Bestimmung des Eigenschaftswerts auf Basis der Auswertung die Wirkung, dass eine Übereinstimmung von Kriterien überprüft werden kann, die an die Produktion gestellt werden. Dies kann konkret eine Überprüfung sein, dass der Kernfaden hinsichtlich des Materials oder Feinheitsgrades der geplanten Produktion der Arbeitsstelle entspricht. Hiermit wird bspw. sichergestellt, dass gegebenenfalls aufgetretene Systemfehler der Textilmaschine hinsichtlich des Transportes und der Zuordnung des Kernfadens vor dem Produktionsstart der Arbeitsstelle erkannt werden können. Es ist möglich, dass bei Feststellung einer fehlerhaften Vorlage der Produktionsprozess nicht gestartet und diese Spule mit dem Kernfaden dem Prozess rückgeführt wird. Eine erneute Anforderung kann ggf. mit einer neuerlichen Prüfung hinsichtlich des Materials oder Feinheitsgrades erfolgen.

Es ist bevorzugt, wenn der Erfassungswert mittels eines Sensors, mehrerer Sensoren oder einer Kraft-Dehnungs-Messvorrichtung, insbesondere direkt oder indirekt, erfasst wurde. Darüber hinaus ist es denkbar, dass das Erfassen des Erfassungswerts, insbesondere mittels eines Sensors, mehrerer Sensoren oder einer Kraft-Dehnungs-Messvorrichtung, Teil des erfindungsgemäßen Verfahrens zur Bestimmung eines Eigenschaftswerts eines Kernfadens ist.

Darüber hinaus kann es sich bei dem Erfassungswert um einen Messwert, also insbesondere einen gemessenen, vorzugsweise direkt oder indirekt gemessenen, Wert, und/oder einen Sensorwert, insbesondere einen mittels eines Sensors gemessenen, vorzugsweise direkt oder indirekt gemessenen, Wert handeln.

Der Erfassungswert kann zum Beispiel in Form von Daten, insbesondere digital oder analog, vorliegen.

Der Eigenschaftswert entspricht vorzugsweise einem Ist-Wert, insbesondere einem Ist-Werkstoff des Kernfadens und/oder einem Ist-Durchmesser des Kernfadens.

Das Empfangen kann darüber hinaus kabelgebunden oder kabellos, insbesondere mittels Funkübertragung, erfolgen. Der Erfassungswert kann in verschlüsselter Form empfangen werden. Hierbei ist es zweckmäßig, wenn der verschlüsselte Erfassungswert vor dem Auswerten entschlüsselt wird. Durch die Verschlüsselung kann die Betriebssicherheit sichergestellt werden.

Das Empfangen kann mittels einer Empfangseinheit durchgeführt werden. Das Auswerten kann hingegen mittels einer Auswerteeinheit ausgeführt werden.

Der Durchmesser des Kernfadens ist vorzugsweise senkrecht zu einer geradlinigen Erstreckungsrichtung und/oder Förderrichtung des Kernfadens messbar. Die Förderrichtung ist vorzugsweise die Richtung, in die der Kernfaden, insbesondere während der Durchführung des erfindungsgemäßen Verfahrens, gefördert wird.

Vorzugsweise wird das erfindungsgemäße Verfahren in Echtzeit und/oder während der Zuführung des Kernfadens zu einer Spinnvorrichtung durchgeführt.

Darüber hinaus kann der Kernfaden dazu bestimmt sein, zur Herstellung eines Spinnfadens, der den Kernfaden umfasst, verwendet zu werden.

Zur Dokumentation kann vorgesehen sein, dass der Erfassungswert abgespeichert wird. Ein derartiger Erfassungswert kann dem später aus dem Kernfaden hergestellten Spinnfaden zugeordnet sein oder zugeordnet werden.

Bei dem Kraft-Dehnungs-Wert handelt es sich vorzugsweise um einen Wert, der einen Zusammenhang zwischen einer Kraft, insbesondere Zugkraft, mit der der Kernfaden beaufschlagt wird und einer sich daraus ergebenden Dehnung beschreibt. Beispielsweise kann eine Zeit oder ein Zeitwert, die bzw. der vergeht, bis der Kernfaden eine spezifische Dehnung oder Längung aufgrund der Kraft, insbesondere Zugkraft, mit der er beaufschlagt wird, erreicht hat, charakteristisch für den Kraft-Dehnungs-Wert sein. Alternativ kann eine Dehnung oder Längung des Kernfadens, die sich aufgrund einer spezifischen Kraft, insbesondere Zugkraft, mit der der Kernfaden beaufschlagt wird, vorzugsweise nach einer spezifischen Zeit oder einem spezifischen Zeitwert ergibt, charakteristisch für den Kraft-Dehnungs-Wert sein. Alternativ kann eine Kraft, insbesondere Zugkraft, mit der der Kernfaden beaufschlagt wird, die für eine spezifische Dehnung oder Längung des Kernfadens notwendig ist, vorzugsweise nach einer spezifischen Zeit oder einem spezifischen Zeitwert, charakteristisch für den Kraft-Dehnungs-Wert sein. Bei der spezifischen Kraft kann es sich um einen konstanten Kraftwert oder einen Kraftwertverlauf handeln. Bei der Dehnung kann es sich um eine konstante Dehnung oder einen Dehnungswertverlauf handeln.

Unter einer spezifischen Kraft ist vorzugsweise eine vorgegebene und/oder hinterlegte Kraft gemeint. Unter einer spezifischen Dehnung ist vorzugsweise eine vorgegebene und/oder hinterlegte Dehnung gemeint. Unter einer spezifischen Zeit ist vorzugsweise eine vorgegebene und/oder hinterlegte Zeit gemeint.

Unter Beaufschlagen des Kernfadens mit einer Kraft, insbesondere Zugkraft, kann grundsätzlich gemeint sein, dass der Kernfaden der Kraft, insbesondere Zugkraft, ausgesetzt ist. Der Faden ist vorzugsweise in Förderrichtung und/oder Erstreckungsrichtung mit der Kraft beaufschlagt.

Ferner können mehrere Erfassungswerte empfangen werden, die jeweils für Kraft-Dehnungs-Werte an verschiedenen Abschnitten des Kernfadens charakteristisch sind. Hierbei ist es bevorzugt, wenn die entsprechenden mindestens einen Erfassungswerte nacheinander, insbesondere in zeitlichem Abstand zueinander, in der Reihenfolge ihrer Erfassung empfangen werden.

Auch kann das Verfahren eine Förderung des Kernfadens, insbesondere zu einer Spinneinrichtung, vorzugsweise einer Spinneinrichtung einer Spinnvorrichtung, umfassen. Darüber hinaus kann das Verfahren durchgeführt werden während ein Spinnfaden hergestellt wird, der den Kernfaden, insbesondere den bereits geprüften Kernfaden, umfasst. Vorzugsweise wird das Verfahren während der Förderung des Kernfadens durchgeführt. Alternativ wird das Verfahren stationär, insbesondere während der Kernfaden förderbewegungsfrei ist, durchgeführt.

Es ist bevorzugt, wenn sich der mindestens eine Erfassungswert auf einen Abschnitt, insbesondere Längenabschnitt, des Kernfadens bezieht. Ferner ist es bevorzugt, wenn das Verfahren für mehrere aneinandergrenzende oder zueinander beabstandete oder alle Abschnitte, insbesondere Längenabschnitte, des Kernfadens durchgeführt wird. Somit ist eine stichprobenartige Untersuchung der Kernfadens oder eine kontinuierliche Untersuchung des Kernfadens realisierbar.

Es ist denkbar, dass das erfindungsgemäße Verfahren durch eine Initialisierung eines Spinnvorgangs oder eines Spinnverfahrens oder durch das Einsetzen einer Kernfadenspule in eine Aufnahme oder Halterung initialisiert wird. Hierbei ist es zweckmäßig, wenn das erfindungsgemäße Verfahren beendet oder unterbrochen wird, wenn der Spinnvorgang oder das Spinnverfahren beendet oder unterbrochen wird. Mit dem Spinnverfahren oder dem Spinnvorgang ist vorzugsweise eine Herstellung eines Spinnfadens, der den Kernfaden, vorzugsweise nach seiner Prüfung oder Untersuchung, umfasst.

Darüber hinaus ist es bevorzugt, wenn das Auswerten einen Abgleich des mindestens einen Erfassungswerts mit mindestens einem Referenzwert, insbesondere aus einer Zuordnungsmatrix, umfasst. Die Zuordnungsmatrix kann eine Mehrzahl von hinterlegten Referenzwerten umfassen. Anstatt einer Zuordnungsmatrix kann auch ein, insbesondere mehrdimensionales, Kennfeld verwendet werden, um vorzugsweise die Referenzwerte bereitzustellen. Die Referenzwerte, die Zuordnungsmatrix oder das Kennfeld sind vorzugsweise hinterlegt, beispielsweise abgespeichert, und/oder vorbestimmt, beispielsweise experimentell oder kalkulatorisch ermittelt. Mittels des Abgleichs ist vorzugsweise ein Vergleich gemeint.

Darüber hinaus ist es bevorzugt, wenn dem mindestens einen Referenzwert ein Eigenschaftswert, insbesondere ein Werkstoff und/oder eine Feinheit, bevorzugterweise ein Durchmesser, eines Referenzkernfadens zugeordnet ist.

Darüber hinaus ist es bevorzugt, wenn der mindestens eine Kraft-Dehnungs-Wert einem Kraft-Dehnungs-Wertverlauf entspricht. Hierbei kann es denkbar sein, dass der Kraft-Dehnungs-Wertverlauf einen stetigen Verlauf aufweist. Außerdem ist es denkbar, dass der Kraft-Dehnungs-Wertverlauf aus mehreren Kraft-Dehnungs-Werten besteht oder mehrere Kraft-Dehnungs-Werte umfasst, wobei die Kraft-Dehnungs-Werte jeweils aufeinanderfolgenden Abschnitten des Kernfadens zugeordnet sind.

Darüber hinaus ist es bevorzugt, wenn der mindestens eine Kraft-Dehnungs-Wert einer Kraft, insbesondere Zugkraft, für eine spezifische Dehnung, insbesondere Längsdehnung, oder einer Dehnung, insbesondere Längsdehnung, für eine spezifische Kraft, insbesondere Zugkraft, entspricht. Die Längsdehnung entspricht vorzugsweise einer Dehnung oder Längung des Kernfadens in Förderrichtung und/oder in Richtung einer geradlinigen Erstreckung des Kernfadens. Unter einer Dehnung oder Längung ist vorzugsweise ein Längenunterschied zwischen einem nicht mit der Kraft beaufschlagten und einem mit der Kraft beaufschlagten Faden zu verstehen. Die Dehnung kann mittels eines Sensors, insbesondere direkt oder indirekt, ermittelt werden. Ein Beispiel für eine indirekte Ermittlung kann ein optischer Sensor sein, der eine Abnahme des Durchmessers aufgrund der Beaufschlagung des Kernfadens mit der Kraft erfasst, wobei diese Abnahme charakteristisch für die Dehnung ist. Ein Beispiel für eine direkte Ermittlung kann ein optischer Sensor sein, der eine Zunahme der Länge eines Abschnitts des Kernfadens aufgrund der Beaufschlagung des Kernfadens mit der Kraft erfasst, wobei diese Zunahme charakteristisch für die Dehnung ist. Grundsätzlich kann unter Dehnung und Längung dasselbe oder das Gleiche verstanden werden. Anstatt einem optischen Sensor kann ein kapazitiver Sensor eingesetzt werden, der vorzugsweise den Durchmesser des Kernfadens bestimmen kann. Die Erfassung des Durchmessers des Kernfadens ist auch mit einem optischen Sensor denkbar. Aufgrund der Dehnung des Kernfadens kommt es zu einer Abnahme oder Reduzierung des Durchmessers des Kernfadens. Diese Abnahme oder Reduzierung des Durchmessers ist charakteristisch für die Dehnung oder Längung des Kernfadens.

Darüber hinaus ist es bevorzugt, wenn der mindestens eine Erfassungswert einem Erfassungswert bei einer Temperierung des Kernfadens auf einen spezifischen Temperaturwert, insbesondere Temperaturwertverlauf oder konstanten Temperaturwert, oder mit einer spezifischen Heizleistung entspricht. Unter der Temperierung ist vorzugsweise ein Heizen oder Aufheizen oder Beheizen des Kernfadens auf einen spezifischen Temperaturwert zu verstehen. Hierfür wird vorzugsweise eine Temperierungsvorrichtung, insbesondere ein Heizer, verwendet. Alternativ zur Temperierung auf einen spezifischen Temperaturwert kann der Kernfaden einer spezifischen Heizleistung ausgesetzt sein. Auch hierfür kann eine Temperierungsvorrichtung, insbesondere ein Heizer, verwendet werden. Vorzugsweise kann die Erfassung des Erfassungswerts initialisiert werden, wenn die Temperatur des Kernfadens einen Temperaturschwellwert erreicht hat. Das Erreichen des Temperaturschwellwerts kann vorzugsweise mit einem Temperatursensor ermittelt werden. Durch das Temperieren des Kernfadens werden Umgebungseinflüsse, wie die Umgebungstemperatur, die Einfluss auf den Erfassungswert haben können, weitestgehend neutralisiert.

Darüber hinaus ist es bevorzugt, wenn das Verfahren ferner umfasst:
- Initialisieren einer Temperierung des Kernfadens, insbesondere auf einen spezifischen Temperaturwert und/oder mit einer spezifischen Heizleistung, und/oder
- Initialisieren einer Beaufschlagung des Kernfadens mit einer Kraft, insbesondere Zugkraft, wobei die Kraft vorzugsweise durch zwei Antriebsmittel zum Fördern des Kernfadens erzeugt wird, wobei eines der Antriebsmittel in Förderrichtung zum Abschnitt angeordnet ist und das weitere Antriebsmittel entgegen der Förderrichtung angeordnet ist, wobei die Antriebsmittel mit einer Differenzgeschwindigkeit zur Erzeugung der Kraft des Kernfadens betrieben werden.

Vorzugsweise folgt das Initialisieren einer Beaufschlagung des Kernfadens mit einer Kraft dem Initialisieren einer Temperierung des Kernfadens zeitlich, insbesondere unmittelbar oder bis der Temperaturschwellwert erreicht ist. Mit anderen Worten kann das Initialisieren der Beaufschlagung mit einer Kraft, vorzugsweise unmittelbar, erfolgen, nachdem der Temperaturschwellwert erreicht ist. Hierdurch werden reproduzierbare Messergebnisse sichergestellt.

Durch die Beaufschlagung des Kernfadens mit der Kraft mittels der zur Förderung des Fadens notwendigen Antriebe kann eine sehr kostengünstige Kraftausübungsvorrichtung geschaffen werden, da keine zusätzlichen Mittel zur Ausbildung der Kraftausübungsvorrichtung notwendig sind. Alternativ kann die Kraftausübungsvorrichtung dadurch ausgebildet sein, dass die Aufnahme oder Halterung für die Kernfadenspule einen konstanten Abziehwiderstand aufweist, der mit einem Antrieb zum Fördern des Kernfadens, insbesondere zum Abziehen des Kernfadens von der Kernfadenspule, derart zusammenwirkt, dass der Kernfaden bei Abziehen von der Kernfadenspule mit einer spezifischen Kraft beaufschlagt und abgezogen wird.

Ebenfalls Gegenstand der Erfindung ist ein Verfahren zum Betrieb einer Spinnvorrichtung, wobei das Verfahren die Verfahrensschritte des erfindungsgemäßen Verfahrens zur Bestimmung eines Eigenschaftswerts eines Kernfadens umfasst, wobei das Verfahren zum Betrieb einer Spinnvorrichtung ferner umfasst, die Spinnvorrichtung auf Basis des bestimmten Eigenschaftswerts zu betreiben. Damit bringt das erfindungsgemäße Verfahren zum Betrieb einer Spinnvorrichtung die gleichen Vorteile mit sich, wie sie ausführlich mit Bezug auf ein erfindungsgemäßes Verfahren zur Bestimmung eines Eigenschaftswerts eines Kernfadens beschrieben worden sind.

Mittels der Spinnvorrichtung kann ein Spinnfaden hergestellt werden, der den Kernfaden umfasst. Die Abschnitte des Kernfadens, die zum Spinnfaden verarbeitet werden oder wurden, sind zuvor mittels des erfindungsgemäßen Verfahrens zur Bestimmung eines Eigenschaftswerts eines Kernfadens untersucht oder geprüft worden. Die Spinnvorrichtung kann vorzugsweise einen Spinnrotor umfassen, der dazu ausgebildet ist, den Kernfaden zusammen mit dem einen Spinnrotorfaden, gebildet aus dem Spinnrotor zugeführten Fasern in dem Spinnrotor zu einem Spinnfaden zu spinnen. Der gesponnene Spinnfaden wird vorzugsweise nach dessen Herstellung auf eine Spule oder Rolle aufgewickelt und/oder in einer dafür vorgesehenen Kanne untergebracht. Die erfassten Eigenschaftswerte werden der Kanne und/oder der Spule oder Rolle mit dem Spinnfaden zugeordnet, sodass insbesondere auch zu einem späteren Zeitpunkt Informationen über die Eigenschaftswerte zum hergestellten Spinnfaden vorhanden sind.

Darüber hinaus ist es bevorzugt, wenn das Verfahren Folgendes umfasst:
- Initialisieren eines Spinnverfahrens mittels des Kernfadens und der Spinnvorrichtung, wenn der mindestens eine Eigenschaftswert des Kernfadens mindestens einem Sollwert entspricht, und/oder
- Verhindern einer Initialisierung eines Spinnverfahrens mittels des Kernfadens und der Spinnvorrichtung, wenn der mindestens eine Eigenschaftswert des Kernfadens von mindestens einem Sollwert oder dem mindestens einen Sollwert abweicht.

Bei dem Spinnverfahren handelt es sich vorzugsweise um ein Spinnverfahren zur Herstellung eines Spinnfadens. Durch das Verhindern der Initialisierung kann ein möglicher Ausschuss des mit dem Kernfaden hergestellten Spinnfadens verhindert werden, da lediglich eine Freigabe der Produktion, also des Spinnverfahrens erfolgt, wenn der Kernfaden den vorgegebenen Kriterien, also Sollwerten, entspricht. Im Fall, dass die Initialisierung des Spinnfahrens verhindert wird, kann eine Nachricht, ein akustisches und/oder optisches und/oder haptisches Signal generiert werden, um, vorzugsweise einen Bediener, darauf hinzuweisen, dass der Kernfaden nicht den Anforderungen genügt. Zusätzlich oder alternativ hierzu kann ein Wechsel des Kernfadens initialisiert werden, wenn ein Initialisieren des Spinnverfahrens verhindert wird, vorzugsweise so lange bis ein Kernfaden vorliegt, dessen Eigenschaftswerte den Sollwerten entsprechen.

Bei dem Sollwert handelt es sich vorzugsweise um einen Soll-Werkstoff des Kernfadens oder einen Soll-Durchmesser, insbesondere eine Soll-Feinheit, des Kernfadens.

Grundsätzlich kann unter einem Material auch ein Werkstoff verstanden werden und umgekehrt.

Ebenfalls Gegenstand der Erfindung ist eine Vorrichtung zur Datenverarbeitung, die Mittel zur Ausführung einer der erfindungsgemäßen Verfahren umfasst. Damit bringt die erfindungsgemäße Vorrichtung die gleichen Vorteile mit sich, wie sie ausführlich mit Bezug auf die erfindungsgemäßen Verfahren beschrieben worden sind.

Bei den Mitteln kann es sich um Programmcode und/oder um konstruktive Elemente und Baugruppen, wie beispielsweise Aktuatoren und/oder Sensoren und/oder Vorrichtungen handeln.

Als die Vorrichtung zur Datenverarbeitung kann ein Computer oder eine Datenverarbeitungsvorrichtung angesehen werden. Ferner kann es sich bei der Vorrichtung zur Datenverarbeitung um ein Steuergerät und/oder eine Auswerteeinheit handeln.

Vorzugsweise kann mittels der Vorrichtung zur Datenverarbeitung der mindestens eine Erfassungswert empfangen werden und/oder der mindestens eine Erfassungswert ausgewertet werden und/oder der Eigenschaftswert bestimmt werden und/oder die Temperierung des Kernfadens initialisiert werden und/oder die Beaufschlagung des Kernfadens mit einer Kraft initialisiert werden und/oder die Spinnvorrichtung auf Basis des bestimmten Eigenschaftswerts betrieben werden und/oder das Spinnverfahren initialisiert werden und/oder die Initialisierung des Spinnverfahrens verhindert werden.

Ebenfalls Gegenstand der Erfindung ist eine Fadensensoreinrichtung, insbesondere Kernfadensensoreinrichtung, für eine Spinnvorrichtung, vorzugsweise umfassend eine erfindungsgemäße Vorrichtung zur Datenverarbeitung. Dabei kann die Fadensensoreinrichtung insbesondere eine Kraft-Dehnungs-Messvorrichtung zur Ermittlung mindestens eines Kraft-Dehnungs-Werts eines Kernfadens und/oder eine Temperierungsvorrichtung, vorzugsweise Heizer, zur Temperierung des Kernfadens aufweisen. Damit bringt die erfindungsgemäße Fadensensoreinrichtung die gleichen Vorteile mit sich, wie sie ausführlich mit Bezug auf die erfindungsgemäße Vorrichtung zur Datenverarbeitung und erfindungsgemäßen Verfahren beschrieben worden sind. Die Fadensensoreinrichtung kann auch als Kernfadenprüfer bezeichnet werden.

Die Kraft-Dehnungs-Messvorrichtung kann einen oder mehrere Sensoren, vorzugsweise zur direkten oder indirekten Messung oder Erfassung der Dehnung oder Längung des Kernfadens, und/oder einen oder mehrere Sensoren zur direkten oder indirekten Messung oder Erfassung der auf den Kernfaden beaufschlagten Kraft, insbesondere Zugkraft, umfassen. Mit anderen Worten umfasst die Kraft-Dehnungs-Messvorrichtung alle Mittel zur Erfassung des Erfassungswerts, insbesondere des Kraft-Dehnungs-Werts. Optional kann die Kraft-Dehnungs-Messvorrichtung auch eine Kraftausübungsvorrichtung zur Beaufschlagung des Kernfadens mit einer Kraft, insbesondere Zugkraft umfassen.

Die Temperierungsvorrichtung, vorzugsweise der Heizer, umfasst einen Temperatursensor zur Erfassung und/oder Messung der Temperatur des Kernfadens oder des Kernfadenabschnitts, der mit der Temperierungsvorrichtung, vorzugsweise dem Heizer, temperiert oder beheizt wird. Vorzugsweise ist die Temperierungsvorrichtung, vorzugsweise der Heizer, in Abhängigkeit des Temperatursensors betreibbar. Auf diese Weise kann die Heizleistung der Temperierungsvorrichtung, vorzugsweise des Heizers, derart geregelt oder gesteuert werden, dass ein Temperaturschwellwert für die Temperatur des Kernfadens zwar erreicht, aber nicht überschritten wird, während der Empfangswert und/oder Kraft-Dehnungs-Wert des Kernfadens ermittelt wird. Der Heizer kann vorzugsweise als Heizwiderstand ausgebildet sein, der insbesondere durch Anlegen einer Spannung erhitzt werden kann. Der Heizwiderstand kann vorzugsweise als PTC ausgebildet sein.

Ebenfalls Gegenstand der Erfindung ist eine Spinnvorrichtung, welche eine erfindungsgemäße Fadensensoreinrichtung aufweist, wobei die Fadensensoreinrichtung einer Spinneinrichtung der Spinnvorrichtung zur Herstellung eines den Kernfaden umfassenden Spinnfadens vorgelagert ist und/oder wobei die Fadensensoreinrichtung eine Kraftausübungsvorrichtung umfasst, um den Kernfaden mit einer Kraft, insbesondere Zugkraft, vorzugsweise während der Ermittlung des mindestens einen Kraft-Dehnungs-Werts, zu beaufschlagen, wobei die Kraftausübungsvorrichtung vorzugsweise in einen Antrieb zur Förderung des Kernfadens zur Spinnvorrichtung integriert ist. Damit bringt die erfindungsgemäße Fadensensoreinrichtung die gleichen Vorteile mit sich, wie sie ausführlich mit Bezug auf die erfindungsgemäße Vorrichtung zur Datenverarbeitung und erfindungsgemäßen Verfahren beschrieben worden sind.

Die Spinnvorrichtung kann vorzugsweise Bestandteil einer Arbeitsstelle einer Textilmaschine zum Spinnen eines Spinnfadens oder einer Spinnmaschine zum Spinnen eines Spinnfadens verstanden werden. Die Textilmaschine kann eine Spinnmaschine wie bspw. eine Luftspinnmaschine oder eine Rotorspinnmaschine sein. Entsprechend kann im Rahmen der Erfindung das Spinnverfahren ein Luftspinnen umfassen, ggf. aber auch ein Open End Spinnen bzw. ein Rotorspinnen, ein Friktionsspinnen oder weitere Spinnverfahren betreffen.

Dass die Fadensensoreinrichtung der Spinneinrichtung vorgelagert ist, bedeutet vorzugsweise, dass ein Kernfaden oder Kernfadenabschnitt, der der Spinneinrichtung zugeführt wird, zunächst die Fadensensoreinrichtung passiert bevor der Kernfaden oder Kernfadenabschnitt zur Spinneinrichtung gelangt.

Die Spinneinrichtung kann vorzugsweise auch als Spinnstelle zum Spinnen des Spinnfadens bezeichnet werden. Bei der Spinnstelle kann es sich vorzugsweise um eine Spinnstelle einer Rotorspinnmaschine oder einer Luftspinnmaschine handeln. Die Kraftausübungsvorrichtung kann auch separat zu einem Antrieb zur Förderung des Kernfadens ausgebildet sein.

Darüber hinaus ist es bevorzugt, wenn die Spinnvorrichtung eine Aufnahme, insbesondere Halterung, für eine den Kernfaden umfassende Kernfadenspule aufweist, wobei die Spinnvorrichtung vorzugsweise eine Auswerteeinheit zur Auswertung des mindestens einen Kraft-Dehnungs-Werts auf Basis eines Abgleichs des mindestens einen Kraft-Dehnungs-Werts mit mindestens einem Referenzwert, insbesondere aus einer Zuordnungsmatrix, umfasst, wobei die Spinnvorrichtung vorzugsweise eine Steuereinheit, insbesondere ein Steuergerät, umfasst, die dazu ausgebildet ist, die Spinnvorrichtung zu betreiben, insbesondere derart, dass ein Spinnverfahren mittels des Kernfadens und der Spinneinrichtung zugelassen wird, wenn die Auswertung mit der Auswerteeinheit ergibt, dass der mindestens eine Kraft-Dehnungs-Wert dem mindestens einen Sollwert entspricht, und/oder ein Spinnverfahren mittels des Kernfadens und der Spinneinrichtung verhindert wird, wenn die Auswertung mit der Auswerteeinheit ergibt, dass der mindestens eine Kraft-Dehnungs-Wert von mindestens einem Referenzwert oder dem mindestens einen Referenzwert abweicht. Hierdurch wird ein Ausschuss, umfassend einen Spinnfaden, der den Qualitätsansprüchen nicht genügt, vermieden. Bei der Steuereinheit kann es sich um ein Steuergerät handeln und umgekehrt. Die Aufnahme kann die Kernfadenspule rotatorisch lagern.

Mit anderen Worten betrifft die erfindungsgemäße Spinnvorrichtung vorzugsweise eine Spinnvorrichtung, umfassend
- eine Halterung zum auswechselbaren Aufnehmen und drehbaren Haltern, insbesondere Lagern, einer Kernfadenspule,
- eine Fadensensoreinrichtung, welche
   - der Halterung nachgelagert ist, und
      dazu ausgelegt ist,
         - das Passieren des Kernfadens optisch oder kapazitiv zu prüfen, und
         - einen Durchmesser des passierenden Kernfadens zu messen,
      oder dazu ausgelegt ist,
         - den Kernfaden einzuspannen,
         - den eingespannten Kernfadenbereich definiert zu erwärmen,
         - den erwärmten Kernfadenbereich mit einer Kraft-Weg-Messzugeinheit definiert zu längen, insbesondere zu dehnen, wobei die Längung, insbesondere Dehnung, und die für die definierte Längung, insbesondere definierte Dehnung, erforderliche Kraft zu ermitteln,
- eine Spinneinrichtung zum Herstellen eines Spinnfadens, der den Kernfaden umfasst,
- eine Auswerteeinrichtung, welche
   - mit der Fadensensoreinrichtung kommunikativ gekoppelt ist, und
      dazu ausgelegt ist,
         - den gemessenen Durchmesser mit einer hinterlegten Zuordnungsmatrix abzugleichen, und
         - basierend auf dem Abgleich einen Feinheitsgrad des Kernfadens zu ermitteln,
      oder dazu ausgelegt ist,
         - die ermittelte Längung, insbesondere Dehnung, und die dafür erforderliche Kraft mit einer hinterlegten Zuordnungsmatrix abzugleichen, und
         - basierend auf dem Abgleich das Material, insbesondere den Werkstoff, des Kernfadens zu ermitteln,
- eine Steuereinheit, welche
   - mit der Auswerteeinrichtung kommunikativ gekoppelt ist,
   - ausgelegt ist, den ermittelten Feinheitsgrad mit einem Soll-Wert oder das ermittelte Material mit einem Soll-Material abzugleichen,
   - ausgelegt ist, den Abgleich als akzeptabel einzustufen,
      wenn das Ergebnis des Abgleichs des Feinheitsgrades einem definierten Wert entspricht oder innerhalb eines definierten Wertebereichs liegt, oder
      wenn das Ergebnis des Abgleichs des Materials einem Soll-Material entspricht, und/oder dazu ausgelegt ist, den Abgleich als inakzeptabel einzustufen,
      wenn das Ergebnis des Abgleichs keinem definierten Wert entspricht oder außerhalb eines definierten Wertebereichs liegt, oder
      wenn das Ergebnis des Abgleichs des Materials nicht einem Soll-Material entspricht,
   - und dazu ausgelegt ist,
      einen produktiven Betrieb der Spinneinrichtung freizugeben, wenn das Ergebnis des Abgleichs als akzeptabel oder als nicht inakzeptabel eingestuft wurde, oder
      einen produktiven Betrieb der Spinneinrichtung zu sperren, wenn das Ergebnis des Abgleichs als inakzeptabel oder als nicht akzeptabel eingestuft wurde,
      wobei der produktive Betrieb ein solcher Betrieb ist, bei welchem die Spinneinrichtung einen Spinnfaden mit dem Kernfaden herstellt.

Jedes der vorangehend mit einem Spiegelstrich oder Aufzählungszeichen angeführten Merkmale kann einzeln oder in beliebiger Kombination mit anderen dieser Merkmale lediglich optional ausgebildet sein.

Ebenfalls Gegenstand der Erfindung ist ein Computerprogramm, insbesondere Computerprogrammprodukt, welches Befehle umfasst, die bei der Ausführung des Computerprogramms durch einen Computer diesen veranlassen, eines der erfindungsgemäßen Verfahren auszuführen.

Damit bringt das erfindungsgemäße Computerprogramm, insbesondere Computerprogrammprodukt, die gleichen Vorteile mit sich, wie sie ausführlich mit Bezug auf ein erfindungsgemäßes Verfahren beschrieben worden sind.

Als der Computer kann die Vorrichtung zur Datenverarbeitung, eine Datenverarbeitungsvorrichtung, bspw. eine der erfindungsgemäßen Vorrichtung, vorgesehen sein, welche das Computerprogramm ausführt. Der Computer kann wenigstens einen Prozessor zur Ausführung des Computerprogramms aufweisen. Auch kann ein nicht-flüchtiger Datenspeicher vorgesehen sein, in welchem das Computerprogramm hinterlegt und von welchem das Computerprogramm durch den Prozessor zur Ausführung ausgelesen werden kann.

Ebenfalls ist es denkbar, dass der Computer zumindest einen integrierten Schaltkreis wie einen Mikroprozessor oder eine Anwendungsspezifische integrierte Schaltung (ASIC) oder ein Anwendungsspezifisches Standardprodukt (ASSP) oder einen digitalen Signalprozessor (DSP) oder einen Field Programmable Gate Array (FPGA) oder dergleichen umfasst. Der Computer kann ferner wenigstens eine Schnittstelle zum Datenaustausch, z. B. eine Ethernet-Schnittstelle oder eine Schnittstelle für LAN (Local Area Network) oder WLAN (Wireless Local Area Network) oder System-on-a-Chip (SoC) oder eine andere Funkschnittstelle wie Bluetooth oder Nahfeldkommunikation (NFC) aufweisen. Ferner kann der Computer als ein oder mehrere Steuergeräte, d. h. auch als ein System aus Steuergeräten, ausgeführt sein. Der Computer kann bspw. auch in einer Cloud und/oder als ein Server vorgesehen sein, um über die Schnittstelle die Datenverarbeitung für eine lokale Anwendung zur Verfügung zu stellen. Auch ist es möglich, dass der Computer als ein mobiles Gerät, wie ein Smartphone, ausgeführt ist.

Das Computerprogramm kann auch bereitgestellt werden, indem es auf einem nicht-flüchtigen Speicher oder einem computerlesbaren Medium abgespeichert ist.

Außerdem ist ein computerlesbares Medium denkbar, auf dem das erfindungsgemäße Computerprogramm abgespeichert ist.

Damit bringt das computerlesbare Medium die gleichen Vorteile mit sich, wie sie ausführlich mit Bezug auf ein erfindungsgemäßes Computerprogramm beschrieben worden sind.

Ebenfalls Gegenstand der Erfindung kann ein computerlesbares Speichermedium sein, welches das erfindungsgemäße Computerprogramm umfasst. Das Speichermedium ist bspw. als ein Datenspeicher wie eine Festplatte und/oder ein nicht-flüchtiger Speicher und/oder eine Speicherkarte ausgebildet. Das Speichermedium kann z. B. in den Computer integriert sein.

Darüber hinaus kann eines der erfindungsgemäßen Verfahren, insbesondere vollständig oder teilweise, als ein computerimplementiertes Verfahren und/oder als ein automatisiertes Verfahren ausgeführt sein.

### Figurenbeschreibung

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein.

Es zeigen:
- Fig. 1: ein Ablaufdiagramm eines Ausführungsbeispiels eines erfindungsgemäßen Verfahrens, und
- Fig. 2: eine schematische Darstellung eines Ausführungsbeispiels einer erfindungsgemäßen Spinnvorrichtung.

In den nachfolgenden Figuren werden für die gleichen technischen Merkmale auch von unterschiedlichen Ausführungsbeispielen die identischen Bezugszeichen verwendet.

Häufig ist es wünschenswert, wenn in einem Spinnverfahren wie einem Rotorspinnen/Open End Spinnen, Luftspinnen oder einem Friktionsspinnen vor dem Produktionsstart eine Prüfung einer Kernfadenvorlage erfolgt. Eine solche Prüfung kann hinsichtlich des Materials des Kernfadens 208a oder eines Feinheitsgrades des Kernfadens 208a durchgeführt werden. Hierbei interagieren automatisierte Prozesse mit den Steuerungen der Textilmaschine, der Arbeitsstelle der Textilmaschine und/oder der Anlage bzw. der gesamten Spinnerei. Es kann vorgesehen sein, dass bei einer fehlerhaften Vorlage der Produktionsprozess nicht gestartet wird und die Spule mit dem Kernfaden 208a dem Prozess rückgeführt wird.

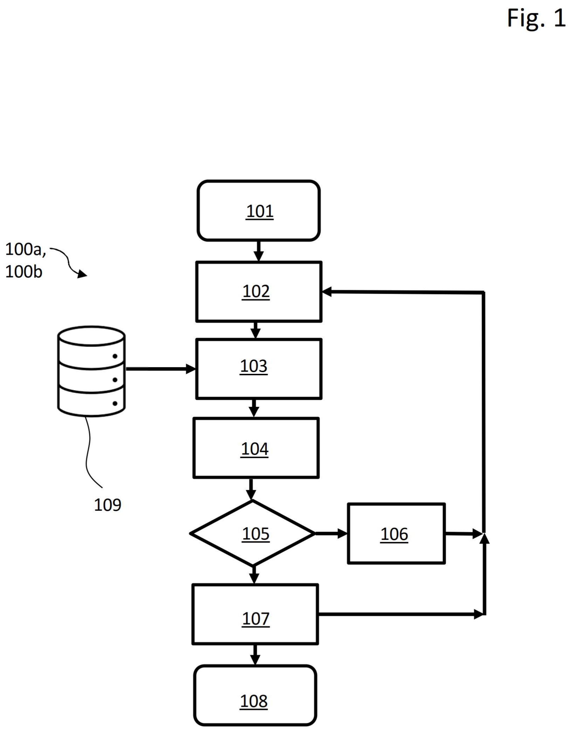

Die Figur 1 zeigt ein Ablaufdiagramm eines Ausführungsbeispiels eines erfindungsgemäßen Verfahrens 100b zum Betrieb einer Spinnvorrichtung 201, welche die Verfahrensschritte eines Ausführungsbeispiels eines erfindungsgemäßen Verfahrens 100a zur Bestimmung eines Eigenschaftswerts eines Kernfadens 208a umfasst. Zunächst erfolgt ein erster Verfahrensschritt 101, der das Initialisieren, also das Starten des Verfahrens 100b umfasst. Daraufhin erfolgt ein zweiter Verfahrensschritt 102, der ein Empfangen von mindestens einem Erfassungswert umfasst, wobei der mindestens eine Erfassungswert für mindestens einen Kraft-Dehnungs-Wert eines Kernfadens 208a spezifisch ist. Der Kernfaden 208a ist dazu vorgesehen, zu einem Spinnfaden 208c verarbeitet zu werden. Der Erfassungswert kann in Form von digitalen Daten funkübertragend oder kabelgebunden, vorzugsweise verschlüsselt, empfangen werden. Im Fall, dass der Erfassungswert verschlüsselt empfangen wird, wird der Erfassungswert nach dem Empfang, aber vor einem dritten Verfahrensschritt 103 entschlüsselt. Der dritte Verfahrensschritt 103 umfasst ein Auswerten des mindestens einen Erfassungswerts, wobei das Auswerten einen Abgleich, insbesondere Vergleich, des mindestens einen Erfassungswerts mit mindestens einem Referenzwert, insbesondere aus einer Zuordnungsmatrix 109, umfasst. Auf Basis der Auswertung erfolgt ein vierter Verfahrensschritt 104, der das Bestimmen eines Eigenschaftswerts, insbesondere Ist-Durchmesser oder Ist-Werkstoff, des Kernfadens 208a umfasst. Der Eigenschaftswert ist vorzugsweise charakteristisch für einen Werkstoff des Kernfadens 208a und/oder einen Durchmesser des Kernfadens 208a. Daraufhin erfolgt der fünfte Verfahrensschritt 105, der ein Betreiben einer Spinnvorrichtung 201 auf Basis des bestimmten Eigenschaftswerts umfasst. Wenn der mindestens eine Eigenschaftswert des Kernfadens 208a von mindestens einem Sollwert, Soll-Werkstoff und/oder Soll-Durchmesser abweicht, erfolgt ein sechster Verfahrensschritt, der eine Initialisierung eines Spinnverfahrens mittels des Kernfadens 208a und der Spinnvorrichtung 201 verhindert. Zusätzlich wird ein Wechsel des Kernfadens 208a initialisiert und das Verfahren, beginnend mit dem zweiten Verfahrensschritt 102, also dem Empfang eines neuen Erfassungswerts des neuen Kernfadens 208a begonnen. Wenn jedoch der mindestens eine Eigenschaftswert des Kernfadens 208a mindestens einem Sollwert entspricht, erfolgt der siebte Verfahrensschritt 107, der ein Initialisieren eines Spinnverfahrens mittels des Kernfadens 208a und einer Spinnvorrichtung 201 umfasst. Nach Beendigung des Spinnverfahrens erfolgt ein achter Verfahrensschritt 108, der ein Beenden des Verfahrens 100b umfasst.

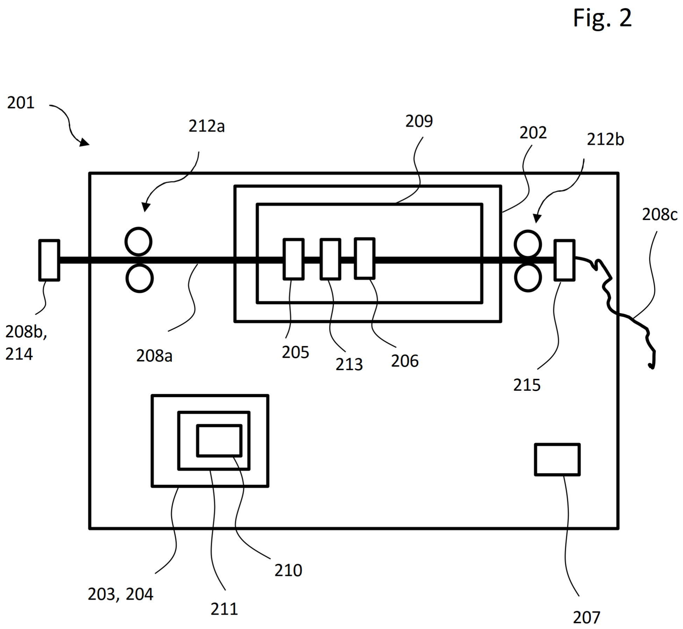

Die Figur 2 zeigt schematisch ein Ausführungsbeispiel einer erfindungsgemäßen Spinnvorrichtung 201. Die Spinnvorrichtung 201 ist dazu ausgebildet, das Verfahren aus Figur 1 auszuführen. Bei der Spinnvorrichtung 201 handelt es sich vorzugsweise um einen Bestandteil einer einen Faden bildenden Textilmaschine wie eine Spinnmaschine zur Herstellung eines Spinnfadens 208c mit einem Kernfaden 208a. Der Kernfaden 208a wird hierfür in Form einer Kernfadenspule 208b einer Aufnahme 214 der Spinnvorrichtung 201 der Spinnvorrichtung 201 bereitgestellt. Die Aufnahme 214 ist als Halterung ausgebildet. Von dort aus kann der Kernfaden 208a mittels eines oder mehrerer Antriebe 212a, 212b zu einer Spinneinrichtung 215 gefördert werden. Die Spinneinrichtung 215 umfasst vorzugsweise einen Spinnrotor zur Herstellung eines Spinnfadens 208c, der den zugeführten Kernfaden 208a umfasst.

Auf dem Förderweg des Kernfadens 208a von der Halterung 214 zur Spinneinrichtung 215 passiert der Kernfaden 208a eine Fadensensoreinrichtung 202, die eine Kraft-Dehnungs-Messvorrichtung 209 umfasst oder als eine solche ausgebildet sein kann. Die Kraft-Dehnungs-Messvorrichtung 209 weist eine Temperiervorrichtung 205 in Gestalt eines Heizers 205 auf, um den Kernfaden 208a auf eine spezifische Temperatur aufzuheizen. Außerdem weist die Kraft-Dehnungs-Messvorrichtung 209 eine Kraftausübungsvorrichtung 206 auf, um den Kernfaden 208a in Längsrichtung mit einer Zugkraft zu beaufschlagen, wenn die spezifische Temperatur des Kernfadens 208a erreicht worden ist, wodurch sich der Kernfaden 208a in Längsrichtung dehnt. Die Längsrichtung entspricht einer geradlinigen Erstreckungsrichtung des Kernfadens 208a und/oder der Förderrichtung des Kernfadens 208a. Ferner weist die Kraft-Dehnungs-Messvorrichtung 209 einen Sensor 213 oder Sensoren 213 auf, um die Dehnung und/oder die Zugkraft zu messen und somit den Erfassungswert zu erfassen, der für mindestens einen Kraft-Dehnungs-Wert spezifisch ist. Die Erfassung des Erfassungswerts kann durchgehend während des Betriebs der Spinnvorrichtung 201 erfolgen. Eine Auswerteeinheit 204 ist dazu ausgebildet, den mindestens einen Erfassungswert zu empfangen und auszuwerten. Die Auswerteeinheit 204 ist vorzugsweise in eine Vorrichtung 203 zur Datenverarbeitung oder einen Computer 203 oder wie in diesem Fall in ein Steuergerät 203 integriert. Das Steuergerät 203 ist dazu ausgebildet, auf Basis des mindestens einen Erfassungswerts einen Eigenschaftswert des Kernfadens 208a zu bestimmen. Der Eigenschaftswert ist charakteristisch für einen Werkstoff, aus dem der Kernfaden 208a besteht, einen Werkstoff oder eine Werkstoffzusammensetzung, die der Kernfaden 208a aufweist und/oder eine Feinheit, insbesondere einen Durchmesser, des Kernfadens 208a. Das Steuergerät 203 ist ferner dazu ausgebildet, die Spinnvorrichtung 201 auf Basis des bestimmten Eigenschaftswerts zu betreiben, insbesondere zu regeln und/oder zu steuern. Das Steuergerät 203 umfasst ein computerlesbares Medium 211, welches als nicht-flüchtiger Speicher ausgebildet umfassend Befehle umfasst, die bei der Ausführung des Computerprogramms 210 durch einen Computer 203, oder in diesem Fall Steuergerät 203, dieses veranlassen, das erfindungsgemäße Verfahren oder die Ausführungsform des Verfahrens aus Figur 1 auszuführen. Darüber hinaus umfasst die Spinnvorrichtung 201 einen Signalgeber 207, um einen Bediener der Spinnvorrichtung 201 akustisch und/oder optisch über eine Verhinderung einer Initialisierung des Spinnverfahrens zu informieren.

Gemäß einem weiteren Ausführungsbeispiel kann die Fadensensoreinrichtung 202 auch als Kernfadenprüfer bezeichnet werden. Dabei kann der Kernfaden 208a auf einer Spule, insbesondere der Kernfadenspule 208b, aufgewunden und einer Produktionsstelle vorgelegt werden. Weiter kann die Spule mit einer Geschwindigkeit *ω***1** angetrieben werden. Der Kernfaden 208a wird insbesondere mithilfe von ein oder mehreren Förderwerken, also insbesondere den voranstehend beschriebenen Antrieben 212a, 212b, mit den Geschwindigkeiten *ω***2**, *ω***3**, *ω**n*** transportiert. Hierdurch wird der Kernfaden 208a dem Spinnprozess mit der Geschwindigkeit *v* zugeführt. Der Kernfadenprüfer 202 kann sich dabei zwischen der Spule, vorzugsweise einer Elastan Faden Spule, und dem "Spinnprozess" befinden. Der Kernfadenprüfer 202 kann über einen optisch oder kapazitiv wirkenden Sensor das Vorhandensein des Kernfadens 208a erkennen. Gleichzeitig oder nachfolgend kann der Sensor auch den Durchmesser des erkannten Kernfadens 208a messen. Im Sensor kann eine Zuordnungsmatrix 109 hinterlegt sein. Dies ermöglicht es, dass mittels der Tabellenwerte der gemessene Durchmesser einem Feinheitsgrad zugeordnet wird. Entspricht dabei der errechnete Wert dem notwendigen Wert, kann der Produktionsstart eingeleitet werden.

Gemäß Ausführungsvarianten der Erfindung kann sich in der Fadensensoreinrichtung 202 bzw. im Kernfadenprüfer ein Thermoelement und/oder eine Kraft - Weg - Zugeinheit befinden. Das Thermoelement kann dazu ausgebildet sein, den eingespannten Kernfadenbereich definiert zu erwärmen. Dabei wird der eingespannte Kernfaden 208a bspw. über ein Heizelement erhitzt, bis zu einer Prüftemperatur, die unterhalb der Glastemperatur von Kunststoffen liegt. Der nun nur noch einseitig eingespannte Faden 208a kann sodann mittels einer Schlaufeneinrichtung um die Länge Δx gelängt werden. Die für diese Längung notwendige Kraft kann ermittelt werden. Es entsteht auf diese Weise ein Kraft-Weg-Zusammenhang.

Im Kernfadenprüfer 202 kann eine Matrix hinterlegt sein, in der typische Kraft - Weg-Daten für Kunststoffe hinterlegt sind. Der aufgenommene Wert kann mit den hinterlegten Werten in der Matrix verglichen und einem Kunststoffmaterial zugeordnet werden. Entspricht der ermittelte Wert dem notwendigen Wert, kann die Produktion starten. Die Überprüfung des Kernfadens 208a kann dabei sicherstellen, dass der Kernfaden 208a hinsichtlich des Materials oder Feinheitsgrades bzw. der geplanten Produktion der Arbeitsstelle entspricht. Hiermit kann ferner sichergestellt werden, dass gegebenenfalls aufgetretene Systemfehler der Maschine hinsichtlich des Transportes und der Zuordnung des Kernfadens 208a vor dem Produktionsstart der Arbeitsstelle erkannt werden.

### Bezugszeichenliste

- 100a: Verfahren
- 100b: Verfahren
- 101: erster Verfahrensschritt
- 102: zweiter Verfahrensschritt
- 103: dritter Verfahrensschritt
- 104: vierter Verfahrensschritt
- 105: fünfter Verfahrensschritt
- 106: sechser Verfahrensschritt
- 107: siebter Verfahrensschritt
- 108: achter Verfahrensschritt
- 109: Zuordnungsmatrix

- 201: Spinnvorrichtung
- 202: Fadensensoreinrichtung, Kernfadenprüfer
- 203: Vorrichtung, Computer, Steuergerät
- 204: Auswerteeinheit
- 205: Temperiervorrichtung, Heizer
- 206: Kraftausübungsvorrichtung
- 207: Signalgeber
- 208a: Kernfaden
- 208b: Kernfadenspule
- 208c: Spinnfaden
- 209: Kraft-Dehnungs-Messvorrichtung
- 210: Computerprogramm
- 211: computerlesbares Medium
- 212a: Antrieb
- 212b: Antrieb
- 213: Sensoren
- 214: Aufnahme, Halterung
- 215: Spinneinrichtung

## Patentansprüche

1. Verfahren (100a) zur Bestimmung eines Eigenschaftswerts mindestens eines Kernfadens (208a) aufweisend die nachfolgenden Schritte:
- Empfangen von mindestens einem Erfassungswert, wobei der mindestens eine Erfassungswert für mindestens einen Kraft-Dehnungs-Wert des Kernfadens (208a) spezifisch ist,
- Auswerten des mindestens einen Erfassungswerts,
- Bestimmen des Eigenschaftswerts, insbesondere des Werkstoffs des Kernfadens (208a) und/oder der Feinheit, bevorzugterweise des Durchmessers, des Kernfadens (208a), auf Basis der Auswertung.

2. Verfahren (100a) nach Anspruch 1, **dadurch gekennzeichnet, dass** das Auswerten (103) einen Abgleich des mindestens einen Erfassungswerts mit mindestens einem Referenzwert, insbesondere aus einer Zuordnungsmatrix (109), umfasst.

3. Verfahren (100a) nach einem der vorhergehenden Ansprüche, **dadurch gekennzeichnet, dass** dem mindestens einen Referenzwert ein Eigenschaftswert, insbesondere ein Werkstoff und/oder eine Feinheit, bevorzugterweise ein Durchmesser, eines Referenzkernfadens zugeordnet ist.

4. Verfahren (100a) nach einem der vorhergehenden Ansprüche, **dadurch gekennzeichnet, dass** der mindestens eine Kraft-Dehnungs-Wert einem Kraft-Dehnungs-Wertverlauf entspricht.

5. Verfahren (100a) nach einem der vorhergehenden Ansprüche, **dadurch gekennzeichnet, dass** der mindestens eine Kraft-Dehnungs-Wert einer Kraft, insbesondere Zugkraft, für eine spezifische Dehnung, insbesondere Längsdehnung, oder einer Dehnung, insbesondere Längsdehnung, für eine spezifische Kraft, insbesondere Zugkraft, entspricht.

6. Verfahren (100a) nach einem der vorhergehenden Ansprüche, **dadurch gekennzeichnet, dass** der mindestens eine Erfassungswert einem Erfassungswert bei einer Temperierung des Kernfadens (208a) auf einen spezifischen Temperaturwert, insbesondere Temperaturwertverlauf oder konstanten Temperaturwert, entspricht.

7. Verfahren (100a) nach einem der vorhergehenden Ansprüche, **dadurch gekennzeichnet, dass** Verfahren ferner umfasst:
- Initialisieren einer Temperierung des Kernfadens (208a), insbesondere auf einen spezifischen Temperaturwert, oder mit einer spezifischen Heizleistung, und/oder
- Initialisieren einer Beaufschlagung des Kernfadens (208a) mit einer Kraft, insbesondere Zugkraft, wobei die Kraft vorzugsweise durch zwei Antriebsmittel zum Fördern des Kernfadens (208a) erzeugt wird, wobei eines der Antriebsmittel in Förderrichtung zum Abschnitt angeordnet ist und das weitere Antriebsmittel entgegen der Förderrichtung angeordnet ist, wobei die Antriebsmittel mit einer Differenzgeschwindigkeit zur Erzeugung der Kraft den Kernfadens (208a) betrieben werden.

8. Verfahren (100b) zum Betrieb einer Spinnvorrichtung (201), wobei das Verfahren (100b) die Verfahrensschritte nach einem der vorhergehenden Ansprüche umfasst, **dadurch gekennzeichnet, dass** die Spinnvorrichtung (201) auf Basis des bestimmten Eigenschaftswerts betrieben wird.

9. Verfahren (100b) nach Anspruch 8, umfassend:
- Initialisieren eines Spinnverfahrens mittels des Kernfadens (208a) und der Spinnvorrichtung (201), wenn der mindestens eine Eigenschaftswert des Kernfadens (208a) mindestens einem Sollwert entspricht, und/oder
- Verhindern einer Initialisierung eines Spinnverfahrens mittels des Kernfadens (208a) und der Spinnvorrichtung (201), wenn der mindestens eine Eigenschaftswert des Kernfadens (208a) von mindestens einem Sollwert oder dem mindestens einen Sollwert abweicht.

10. Vorrichtung (203) zur Datenverarbeitung, die Mittel zur Ausführung eines Verfahrens nach einem der Ansprüche 1 bis 9 umfasst.

11. Fadensensoreinrichtung (202), insbesondere Kernfadensensoreinrichtung, für eine Spinnvorrichtung (201), umfassend die Vorrichtung (203) gemäß Anspruch 10, insbesondere **dadurch gekennzeichnet, dass** die Fadensensoreinrichtung (202) eine Kraft-Dehnungs-Messvorrichtung (209) zur Ermittlung mindestens eines Kraft-Dehnungs-Werts eines Kernfadens (208a) und/oder
eine Temperierungsvorrichtung (205), vorzugsweise Heizer (205), zur Temperierung des Kernfadens (208a) aufweist.

12. Spinnvorrichtung (201), aufweisend eine Fadensensoreinrichtung (202) nach Anspruch 11, wobei die Fadensensoreinrichtung (202) einer Spinneinrichtung (215) der Spinnvorrichtung (201) zur Herstellung eines den Kernfaden (208a) umfassenden Spinnfadens (208c) vorgelagert ist und/oder wobei die Fadensensoreinrichtung (202) eine Kraftausübungsvorrichtung (206) umfasst, um den Kernfaden (208a) mit einer Kraft, insbesondere Zugkraft, vorzugsweise während der Ermittlung des mindestens einen Kraft-Dehnungs-Werts, zu beaufschlagen, wobei die Kraftausübungsvorrichtung (206) vorzugsweise in einen Antrieb (212a, 212b) zur Förderung des Kernfadens (208a) zur Spinneinrichtung (215) integriert ist.

13. Spinnvorrichtung (201) nach Anspruch 12, **dadurch gekennzeichnet, dass** die Spinnvorrichtung (201) eine Aufnahme (214), insbesondere Halterung (214), für eine den Kernfaden (208a) umfassende Kernfadenspule (208b) aufweist,
wobei die Spinnvorrichtung (201) vorzugsweise eine Auswerteeinheit (204) zur Auswertung des mindestens einen Kraft-Dehnungs-Werts auf Basis eines Abgleichs des mindestens einen Kraft-Dehnungs-Werts mit mindestens einem Referenzwert, insbesondere aus einer Zuordnungsmatrix (109), umfasst, wobei die Spinnvorrichtung (201) vorzugsweise eine Steuereinheit, insbesondere ein Steuergerät (203), umfasst, die dazu ausgebildet ist, die Spinnvorrichtung (201) zu betreiben, insbesondere derart, dass ein Spinnverfahren mittels des Kernfadens (208a) und der Spinneinrichtung (215) zugelassen wird, wenn die Auswertung mit der Auswerteeinheit (204) ergibt, dass der mindestens eine Kraft-Dehnungs-Wert dem mindestens einen Sollwert entspricht, und/oder ein Spinnverfahren mittels des Kernfadens (208a) und der Spinneinrichtung (215) verhindert wird, wenn die Auswertung mit der Auswerteeinheit (204) ergibt, dass der mindestens eine Kraft-Dehnungs-Wert von mindestens einem Referenzwert oder dem mindestens einen Referenzwert abweicht.

14. Spinnvorrichtung (201), umfassend
- eine Halterung zum auswechselbaren Aufnehmen und drehbaren Haltern, insbesondere Lagern, einer Kernfadenspule (208b),
- eine Fadensensoreinrichtung (202) nach Anspruch **11,** welche
- der Halterung nachgelagert ist, und
dazu ausgelegt ist,
- das Passieren des Kernfadens (208a) optisch oder kapazitiv zu prüfen, und
- einen Durchmesser des passierenden Kernfadens (208a) zu messen,
oder dazu ausgelegt ist,
- den Kernfaden (208a) einzuspannen,
- den eingespannten Kernfadenbereich definiert zu erwärmen,
- den erwärmten Kernfadenbereich mit einer Kraft-Weg-Messzugeinheit definiert zu längen, insbesondere zu dehnen, wobei die Längung, insbesondere Dehnung, und die für die definierte Längung, insbesondere definierte Dehnung, erforderliche Kraft zu ermitteln,
- eine Spinneinrichtung (215) zum Herstellen eines Spinnfadens (208c), der den Kernfaden (208a) umfasst,
- eine Auswerteeinrichtung, welche
- mit der Fadensensoreinrichtung (202) kommunikativ gekoppelt ist, und dazu ausgelegt ist,
- den gemessenen Durchmesser mit einer hinterlegten Zuordnungsmatrix (109) abzugleichen, und
- basierend auf dem Abgleich einen Feinheitsgrad des Kernfadens (208a) zu ermitteln,
oder dazu ausgelegt ist,
- die ermittelte Längung, insbesondere Dehnung, und die dafür erforderliche Kraft mit einer hinterlegten Zuordnungsmatrix (109) abzugleichen, und
- basierend auf dem Abgleich das Material, insbesondere den Werkstoff, des Kernfadens (208a) zu ermitteln,
- eine Steuereinheit, welche
- mit der Auswerteeinrichtung kommunikativ gekoppelt ist,
- ausgelegt ist, den ermittelten Feinheitsgrad mit einem Soll-Wert oder das ermittelte Material mit einem Soll-Material abzugleichen,
- ausgelegt ist, den Abgleich als akzeptabel einzustufen,
wenn das Ergebnis des Abgleichs des Feinheitsgrades einem definierten Wert entspricht oder innerhalb eines definierten Wertebereichs liegt, oder
wenn das Ergebnis des Abgleichs des Materials einem Soll-Material entspricht, und/oder dazu ausgelegt ist, den Abgleich als inakzeptabel einzustufen,
wenn das Ergebnis des Abgleichs keinem definierten Wert entspricht oder außerhalb eines definierten Wertebereichs liegt, oder
wenn das Ergebnis des Abgleichs des Materials nicht einem Soll-Material entspricht,
- und dazu ausgelegt ist,
einen produktiven Betrieb der Spinneinrichtung (215) freizugeben, wenn das Ergebnis des Abgleichs als akzeptabel oder als nicht inakzeptabel eingestuft wurde, oder
einen produktiven Betrieb der Spinneinrichtung (215) zu sperren, wenn das Ergebnis des Abgleichs als inakzeptabel oder als nicht akzeptabel eingestuft wurde,
wobei der produktive Betrieb ein solcher Betrieb ist, bei welchem die Spinneinrichtung (215) einen Spinnfaden (208c) mit dem Kernfaden (208a) herstellt.

15. Computerprogramm (210), umfassend Befehle, die bei der Ausführung des Computerprogramms (210) durch einen Computer (203) diesen veranlassen, das Verfahren (100a, 100b) nach einem der Ansprüche 1 bis 9 auszuführen.
